# EUROPEAN PATENT SPECIFICATION

(11) **EP 3 737 440 B1**
(45) Date of publication and mention of the grant of the patent: **10.05.2023**
(21) Application number: 18834307.3
(22) Date of filing: 28.12.2018
(51) Int. Cl.: A61M 1/14, A61M 1/16, A61M 1/28

(54) **DISPOSABLE FLUID CIRCUIT WITH THERMOCHROMIC INDICATOR**
EINWEGFLUIDKREISLAUF MIT THERMOCHROMER ANZEIGE
CIRCUIT DE FLUIDE JETABLE AVEC INDICATEUR THERMOCHROMIQUE

(30) Priority: 12.01.2018 US 201815869400
(43) Date of publication of application: 18.11.2020
(73) Proprietor: Fresenius Medical Care Holdings, Inc., Waltham, MA 02451-1457 (US)
(72) Inventor: SCHMIDT, Daniel, Singapore 138687 (SG); JAMES, Philip Scott, Orinda, California 94563 (US); EGLEY, Bert D., Walnut Creek, California 94598 (US)
(74) Representative: Fish & Richardson P.C.
(86) International application number: PCT/US2018/067835
(87) International publication number: WO 2019/139788

(56) References cited:
- US-A- 4 568 330
- US-A- 5 542 919
- US-A1- 2011 092 775
- US-A1- 2016 242 957

## Description

### TECHNICAL FIELD

This disclosure relates to a disposable fluid circuit with a thermochromic indicator.

### BACKGROUND

Dialysis is a treatment used to support a patient with insufficient renal function. The two principal dialysis methods are hemodialysis and peritoneal dialysis.

During hemodialysis ("HD"), the patient's blood is passed through a dialyzer of a dialysis machine while also passing a dialysis solution or dialysate through the dialyzer. A semipermeable membrane in the dialyzer separates the blood from the dialysate within the dialyzer and allows diffusion and osmosis exchanges to occur between the dialysate and the blood flow. These exchanges across the membrane result in the removal of waste products and toxins.

During peritoneal dialysis ("PD"), a patient's peritoneal cavity is periodically infused with a sterile aqueous solution, referred to as a PD solution or dialysate. The membranous lining of the patient's peritoneum acts as a natural semi-permeable membrane that allows diffusion and osmosis exchange to take place between the solution and the blood stream. These exchanges across the patient's peritoneum result in the removal of waste products.

Hemodialysis treatments typically occur several times a week in a clinic or home environment, whereas peritoneal dialysis treatments occur several times a day and are typically completed in a home environment (e.g., overnight while a patient is asleep).

US 5,542,919 describes a peritoneal dialysis device that operates under pressure monitoring volumetrically in the filling phase and pressure-controlled in the emptying phase, and determines the volume of dialysis fluid introduced or discharged from the number of balancing chamber strokes and the ultrafiltration volume from the difference.

US 2011/0092775 describes an expandable surgical retractor that is insertable into a surgical corridor and expanded to a desired size and shape. Cooling allows the retractor to maintain the expanded characteristic.

US 2016/0242957 describes thermal contrast therapy devices, treatment methods for providing thermal contrast therapy, and systems for providing and managing thermal contrast therapy treatments.

US 4,568,330 describes a cardioplegia system in which cardioplegia medication or a mixture of arterial blood and medication is delivered to the heart of a patient undergoing open heart surgery.

### SUMMARY

The invention is defined by the features of independent claim 1. During dialysis, fluids (e.g., dialysate and blood) flow through disposable fluid circuits. While the disposable nature of the fluid circuits helps to reduce contamination risks for a patient, it quickly becomes prohibitively expensive to include electronics (e.g., sensors) in these disposable fluid circuits. However, monitoring and controlling the temperature of fluid flowing through these circuits is vital to an effective dialysis treatment. The fluid circuits described herein present an elegant and cost-effective approach to temperature control by incorporating thermochromic indicators into a traditional disposable fluid circuit. In some implementations, the changes to the thermochromic material may be sensed using an optical sensor and a processor to detect and interpret data. In other implementations, the color change may be visible to a patient, nurse or caregiver to give an indication of the temperature range of a medical fluid flowing through the circuit. Temperature control components, (e.g., a fluid heater), can then be managed based on a detected color change.

Implementations can include one or more of the following features.

In one aspect of the invention, a dialysis tubing set includes at least one fluid line configured to connect a dialysis fluid source and a pump of a dialysis machine; and a flow chamber connected to the at least one fluid line, the flow chamber having a thermochromic material configured to change color when exposed to a specified temperature range. The thermochromic material is configured as an insert and is positioned within the at least one fluid line.

In a related example, a dialysis cassette includes a rigid base defining a recessed region; a flexible membrane attached to the base in a manner such that the flexible membrane, when pressed against the base, cooperates with a portion of the base defining the recessed region to form a pump chamber having an inlet and an outlet port; a thermochromic film attached to the flexible membrane and configured to change color when exposed to a specified temperature range; and tubing connectors positioned along an edge of the cassette, the tubing connectors being configured to attach to corresponding connectors of a dialysis machine.

In yet another aspect of the invention, a dialysis system includes a dialysis machine having an optical sensor, and the disposable tubing set described above. The flow chamber is aligned with the optical sensor.

In some implementations, the thermochromic material is configured to remain visible to a user.

In certain implementations, the tubing set is configured to be aligned with an optical sensor of a dialysis machine.

In some implementations, the optical sensor is a color sensor.

In certain implementations, the thermochromic material includes at least two thermochromic elements each configured to change color at a different temperature.

In some implementations, each thermochromic element is positioned on a different side of the flow chamber.

In certain implementations, a first thermochromic component is positioned above a second thermochromic component.

In certain implementations, the at least one fluid line includes a first fluid path through a central channel of the insert and a second fluid path between the insert and the at least one fluid line.

In some implementations, the specified temperature range is between 35-39 °C.

In certain implementations, the thermochromic material is positioned along an external surface of the flow chamber.

In some implementations, the dialysis machine is a hemodialysis machine.

In certain implementations, the dialysis machine is a peritoneal dialysis machine.

In some implementations, the thermochromic film is co-molded with the flexible membrane.

In certain implementations, the thermochromic film covers at least 75% of an exterior surface area of the flexible membrane.

In some implementations, the system further includes further a light source positioned to align with the flow chamber of the disposable tubing set.

In certain implementations, the flow chamber is positioned outside the dialysis machine.

In some implementations, the method further includes adjusting the temperature of the flowing fluid based on the determined temperature.

In certain implementations, the method further includes directing light transmitted through the thermochromic film and detecting a color change of the thermochromic material in response to the light.

Embodiments can include one or more advantages.

The tubing sets and flow chambers described herein are designed to be used in medical systems (e.g., hemodialysis and peritoneal dialysis systems). These tubing sets and flow chambers can increase patient comfort and treatment compliance by incorporating a thermochromic material that provides a visible indication of the temperature of the fluid flowing through the tubing set. These tubing sets and chambers are versatile and can work across different medical systems while also remaining affordable for patients because, for example, no electronic component or sensor is added to the disposable fluid line set. What is more, these tubing sets and flow chambers can additionally or alternatively be used to verify a dialysis cassette or tubing set is and remains correctly attached to a dialysis machine.

In the heater unit of an HD machine, or along any flow path, the thermochromic material can be used to display a color change for the correct temperature, a temperature that is too high, or temperature that is not hot enough. By setting the range (set point) at which the thermochromic material change can be used for one of the three cases.

The details of one or more implementations are set forth in the accompanying drawings and the description below. Other aspects, features, and advantages will be apparent from the description and drawings, and from the claims.

### DESCRIPTION OF DRAWINGS

FIG. 1 is a perspective view of a peritoneal dialysis (PD) machine including a fluid line set connected to the machine.
FIG. 2 is a schematic illustration of the PD machine of FIG. 1 and a portion of a fluid circuit including a fluid line set.
FIG. 3 is a schematic illustration of the flow chamber and a detection system.
FIG. 4 is a schematic illustration of a flow chamber with an attached or integrated thermochromic component and a corresponding detection system.
FIG. 5 is a schematic illustration of the system including multiple thermochromic surfaces each tuned to a different temperature set point.
FIG. 6 is an enlarged view of a fluid line set compartment of the PD machine of FIG. 1 showing a fluid line set with a thermochromic material attached to the PD machine.
FIG. 7 is a schematic illustration of a light source directing light through a flow chamber having different surface materials and a corresponding detection system.
FIG. 8 is a schematic illustration of a fluid line having a thermochromic insert.
FIG. 9 is a flow chart illustrating a method of performing dialysis using a thermochromic component.

### DETAILED DESCRIPTION

The present specification describes dialysis systems and disposable fluid line sets incorporating thermochromic elements. These thermochromic elements alone or in combination with durable portions of a dialysis machine provide a relatively low-cost and desirable feature that can help promote dialysis treatments at specified temperatures and help improve the effectiveness of dialysis treatments. Although peritoneal dialysis (PD) systems and cassette based fluid line sets are principally discussed herein, it is noted that the concepts described herein may be used with other types of medical devices and/or dialysis systems, including, for example, hemodialysis (HD) systems, HD fluid line sets, and non-cassette based fluid line sets.

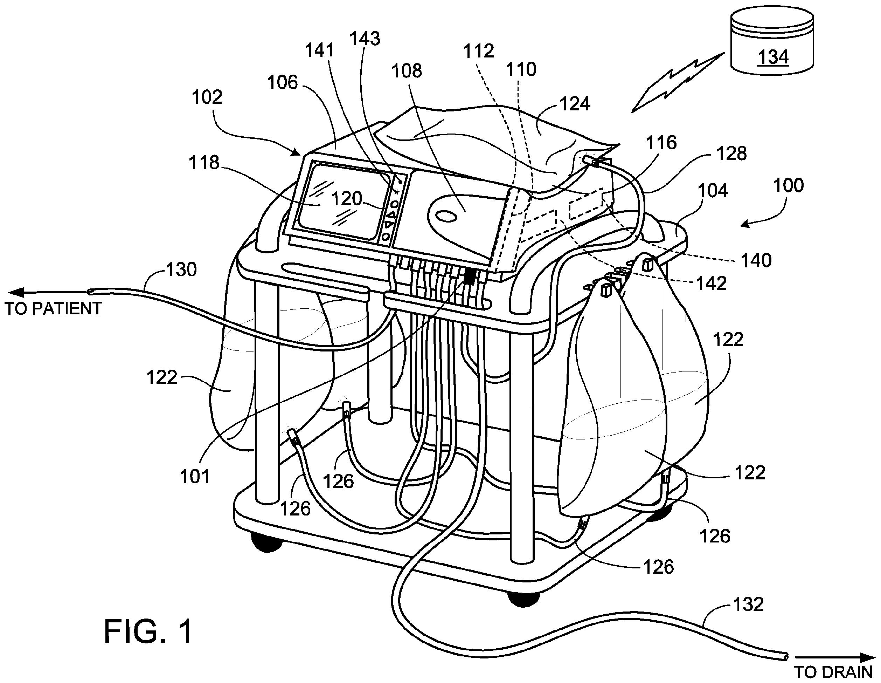

FIG. 1 is a schematic illustration showing an example of a PD system 100 having a system of temperature measurement and control in which the temperature of the medical fluid being conveyed through a medical device is indirectly measured and controlled via sensed color change of an injected molded or extruded thermochromic flow chamber.

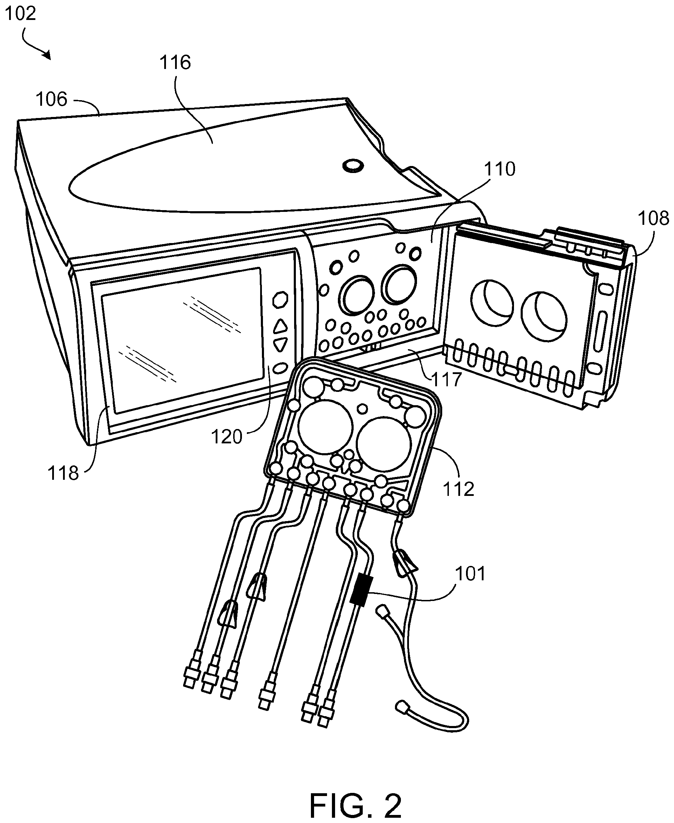

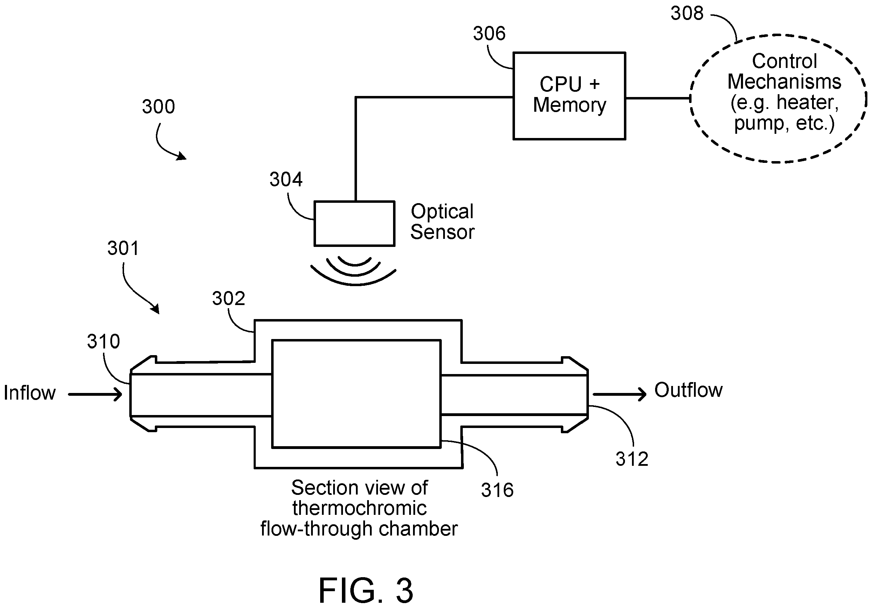

The PD system 100 includes a PD machine (also referred to as a PD cycler) 102 seated on a cart 104, a housing 106, a door 108, and a cassette interface 110 that contacts a disposable PD cassette 112 when the cassette 112 is disposed within a cassette compartment 117 formed between the cassette interface 110 and the closed door 108. The PD system 100 can also include a remote database 134 and one or more controls 141 and 143. FIG. 2 shows the PD machine 102 with the door 108 open and a portion of a fluid circuit including the cassette 112. As described for FIG. 1, the cassette 112 is placed into the cassette compartment 117 between the door 108 and the cassette interface 110. FIG. 1 and FIG. 2 include a representative flow chamber 101 through which fluids pass (e.g., dialysate passes between the cassette 112 and the heater bag 124 through the flow chamber 101 during use). Various implementations of a flow chambers are discussed throughout the specification and shown in, for example, FIGS. 3, 4, and 5.

Referring to FIG. 1, a heater tray 116 is positioned on top of the housing 106. The heater tray 116 is sized and shaped to accommodate a bag of dialysate (e.g., a 5-liter bag of dialysate). The PD machine 102 also includes a user interface such as a touch screen display 118 and additional control buttons 120 that can be operated by a user (e.g., a caregiver or a patient) to allow, for example, set up, initiation, and/or termination of a PD treatment.

Dialysate bags 122 are suspended from fingers on the sides of the cart 104, and a heater bag 124 is positioned in the heater tray 116. The dialysate bags 122 and the heater bag 124 are connected to the cassette 112 via dialysate bag lines 126 and a heater bag line 128, respectively. The dialysate bag lines 126 can be used to pass dialysate from dialysate bags 122 to the cassette 112 during use, and the heater bag line 128 can be used to pass dialysate back and forth between the cassette 112 and the heater bag 124 during use.

In addition, a patient line 130 and a drain line 132 are connected to the cassette 112. The patient line 130 can be connected to a patient's abdomen via a catheter and can be used to pass dialysate back and forth between the cassette 112 and the patient's peritoneal cavity during use. The drain line 132 can be connected to a drain or drain receptacle and can be used to pass dialysate from the cassette 112 to the drain or drain receptacle during use.

The PD machine 102 includes a control unit 140 (e.g. a processor) and one or more sensors, e.g., a color sensor. The PD machine 102 also includes a color sensor (not shown) that is positioned adjacent to at least a portion of the flow chamber 101. The control unit 140 can receive signals from and transmit signals to the touch screen display 118 and/or the control panel 120, and the color sensor (not shown), and the various other components of the PD system 100.

FIG. 3 is a schematic illustration of a system 300 including a flow chamber 301, an optical sensor 304, a processor 306, and a control mechanism 308. During use, fluid enters the flow chamber 301 at an inlet 310 and exits the flow chamber 301 at an outlet 312. A chamber 316 is positioned between the inlet 310 and the outlet 312. A thermochromic layer 302 surrounds the inlet 310, the chamber 316, and the outlet 312. As shown in FIG. 3, the chamber 316 of the flow chamber 301 aligns with an optical sensor 304 of the system 300. The optical sensor 304 is configured to detect color changes in the thermochromic layer 302 and send this information to the processor 306. Any suitable optical sensor can be used (e.g., a TCS34725 color sensor). In some examples, the optical sensor can view a small area (e.g., 1mm by 1mm). In some examples, a low cost thermochromic flow chamber can have an internal diameter ranging from about 1.5 mm (0.06 inches) to 2.5 cm (1 inch), e.g., 2.5 to 7.6 mm (0.1 to 0.3 inches).

The thermochromic layer may be formed of a temperature sensitive polymer that reversibly changes color when exposed to specific temperature ranges. The thermochromic layer may be used in injection molding plastics including: polypropylene, polystyrene, polyethylene, ABS, PVC, and PC. Activation temperatures can range from -10 °C to 69 °C. Suitable materials are discussed in Seeboth, et al. "First Examples of Non-Toxic Thermochromic Polymer Material - Based on a Novel Mechanism" J. Mater. Chem. C, 2013,1, 2811-2816, the contents of which are incorporated by reference in their entirety.

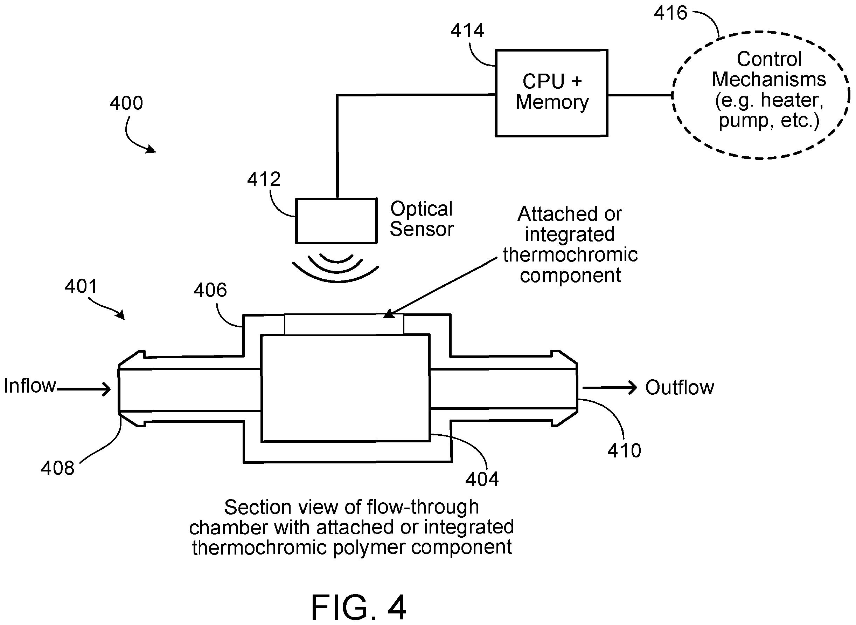

FIG. 4 is a schematic illustration of a system 400 having a flow chamber 401 with a thermochromic element 406 attached to a chamber 404 of the flow chamber 401. During use, fluid enters the flow chamber at an inlet 408 and exits the flow chamber 401 at an outlet 410. The chamber 404 is positioned between the inlet 408 and the outlet 410. The thermochromic element 406 aligns with the optical sensor 412 of the system 400. The optical sensor 412 is configured to detect color changes of the thermochromic element 406 and communicate this information to the processor 414 and/or a control mechanisms 416. The control mechanisms 416 can include, e.g., heaters and/or pumps. Any suitable optical sensor can be used (e.g., a TCS34725 color sensor).

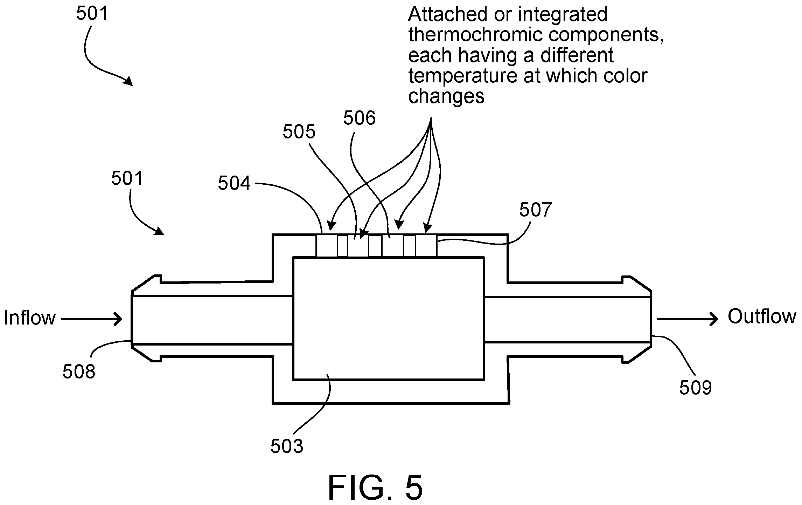

FIG. 5 is a schematic illustration of a system 500 including multiple thermochromic elements (e.g., first thermochromic element 504, second thermochromic element 505, a third thermochromic element 506, and a fourth thermochromic element 507) each tuned to a different temperature set point. During use, fluid enters the flow chamber 501 at an inlet 508 and exits the flow chamber 501 at an outlet 509. A chamber 503 is positioned between the inlet 508 and the outlet 509 and in contact with the thermochromic elements 504, 505, 506, and 507. In some examples, the thermochromic elements 504-507 are arranged in a specific order to detect a specific temperature range (e.g., 30 °C to 41 °C).

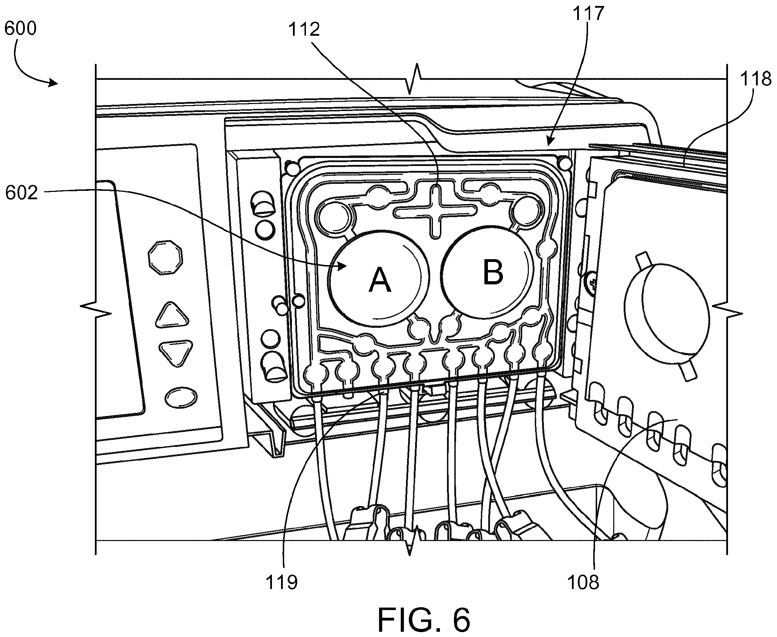

FIG. 6 shows a partial view 600 of a cassette 112 positioned in the cassette compartment 117 of a PD machine. The cassette 112 includes a thermochromic thin-film material 602. The thermochromic thin-film material 602 can respond more quickly to temperature changes as compared to thicker plastic components.

The cassette 112 can be manufactured using suitable molding techniques. As part of the molding process, the thermochromic material is co-molded with a component of the cassette 112 or another portion of a disposable fluid line set. For example, thermochromic components could be over molded or bonded to a substrate by ultrasonic welding or using adhesives.

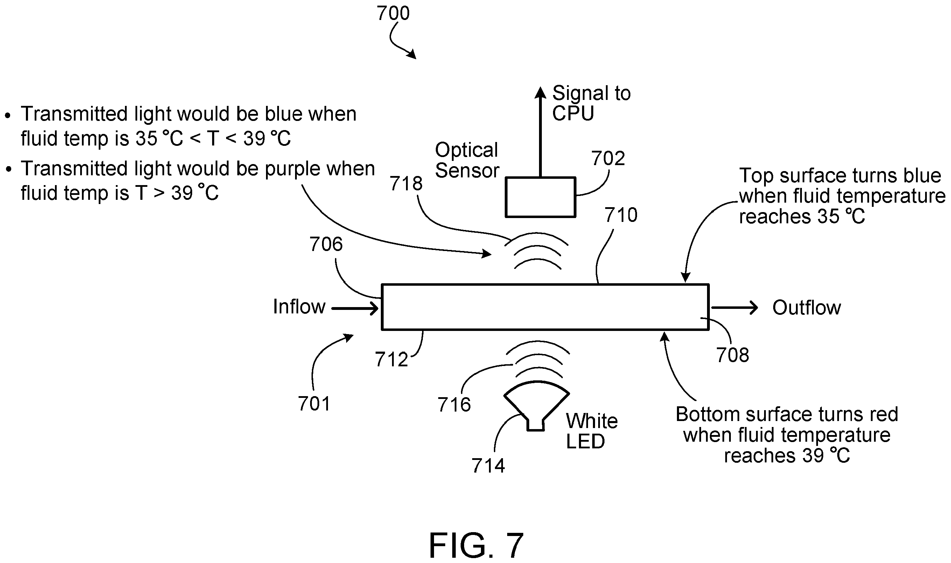

FIG. 7 is a schematic illustration of a system 700 having a fluid path 701 positioned between a light source 714 and an optical sensor 702. The light source 714 can be a white LED or other suitable light source. The fluid pathway 701 includes a first thermochromic layer 712 positioned across from a second thermochromic layer 710. The first thermochromic layer 712 includes a material that turns red when fluid in the fluid path reaches 39 °C. The second thermochromic layer 710 includes a material that turns blue when the fluid temperature reaches 35 °C.

During use, fluid flows along the fluid path 701 and through an inlet 706 and an outlet 708. Light 716 from the light source 714 will pass through the first thermochromic layer 712 and the second thermochromic layer 710. Information representing transmitted light 718 is detected by the optical sensor 702. Based on the arrangement of the thermochromic layers, the transmitted light 718 is red if the fluid temperature reaches 39 °C, blue if the fluid temperature is between 35 °C and 39 °C, and purple if the fluid temperature exceeds 39 °C.

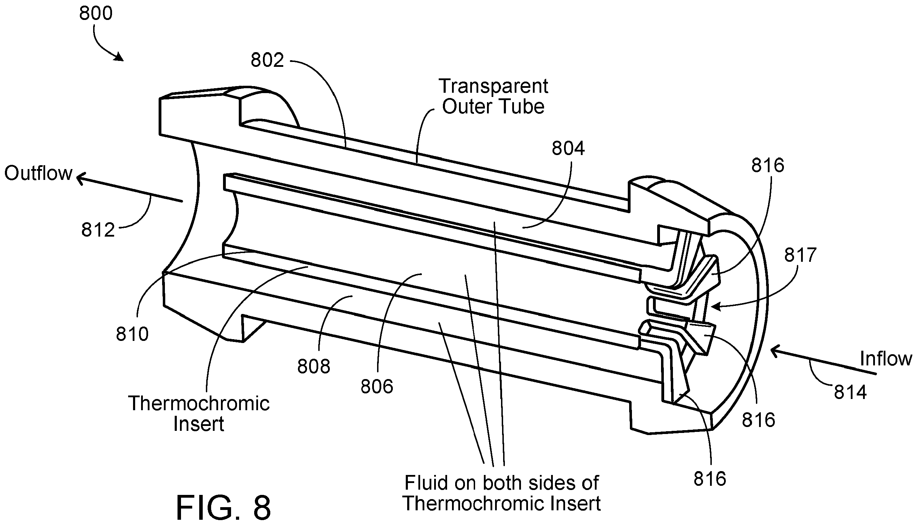

FIG. 8 is a cutaway view 800 of a thermochromic insert 810 positioned within a transparent outer tube 802. Like the flow chambers discussed elsewhere, the thermochromic insert 810 includes a thermochromic material or component configured to change color when exposed to specified temperature ranges. During use, an inflow 814 enters the outer tube 802 and flows on both sides 804, 808 of the insert and through a central channel 806 of the insert 810 before reaching the outlet 812. In this example, fluid is diverted between one or more tabs 816 of the thermochromic insert 810. The thermochromic insert is positioned and centered within the outer tube 802 by placing the one or more tabs on a flange 817 of the outer tube 802. By placing the thermochromic component directly within the fluid flow, the thermochromic insert 810 can quickly respond to temperature changes as there is no intermediate material impeding heat transfer.

### Methods of Use not falling under the claimed invention

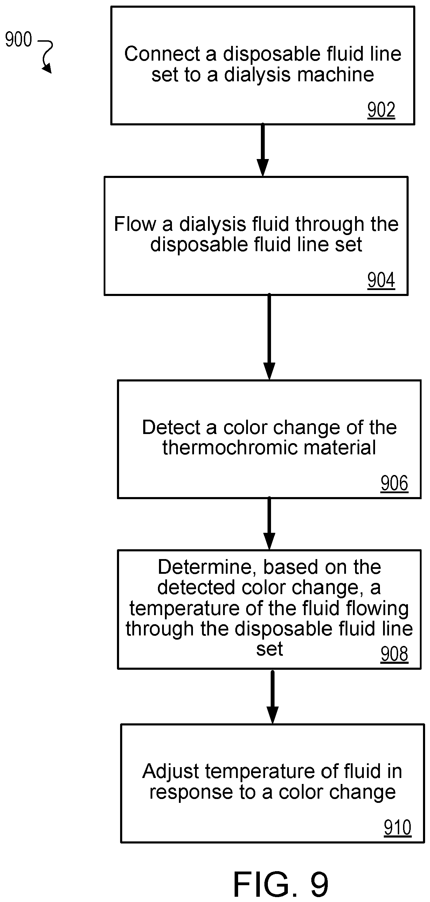

FIG. 9 is a flow chart illustrating a method 900 of performing dialysis using a thermochromic component. To prepare for treatment, a user connects 902 a disposable fluid line set (e.g., the cassette 112) to a dialysis machine. Then, a dialysis fluid flows 904 through the disposable fluid line set. For example, from a fluid source (e.g., a dialysate source) through the dialysis machine and to a patient. As the thermochromic component is exposed to the temperature of the fluid, any color change is detected 906. The detection can be automated when the machine includes suitable optical sensors (as described above) or a color change can be manually, e.g., visually, detected by a patient or user. Based on any detected color change, the machine and/or the user can determine 908 the temperature of the fluid flowing through the disposable fluid line set. If the temperature deviates from an acceptable or expected, the machine and/or user can adjust 910 the temperature of the fluid based on the color change.

### Alternative implementations

The examples described herein can be implemented in a variety of ways without departing from the scope of the specification.

The thermochromic element 406 is integrated with the flow chamber 401. When integrated with the flow chamber 401, the thermochromic element is in direct contact with the medium being monitored which can yield a faster and/or more accurate response to the temperature change. In addition, manufacturing efficiencies can be realized.

While the blue surface is generally shown adjacent to the optical sensor and the red surface is shown adjacent to the light source 714 in FIG. 7, other implementations are possible. For example, the red surface can be adjacent to the optical sensor 702, and the blue surface can be adjacent to the light source 714.

Elements of different implementations described herein may be combined to form other implementations not specifically set forth above. Elements may be left out of the structures described herein without adversely affecting their operation. Furthermore, various separate elements may be combined into one or more individual elements to perform the functions described herein.

Various embodiments discussed herein may be combined with each other in appropriate combinations with the system described herein. Additionally, in some instances, the order of steps in a method may be modified, where appropriate. Further, various aspects of the systems described herein may be implemented using software, hardware, a combination of software and hardware and/or other computer-implemented modules or devices having the described features and performing the described functions.

Software implementations of aspects of the system described herein may include executable code that is stored in a computer-readable medium and executed by one or more processors. The computer-readable medium may include volatile memory and/or non-volatile memory, and may include, for example, a computer hard drive, ROM, RAM, flash memory, portable computer storage media such as a CD-ROM, a DVD-ROM, a flash drive and/or other drive with, for example, a universal serial bus (USB) interface, and/or any other appropriate tangible or non-transitory computer-readable medium or computer memory on which executable code may be stored and executed by a processor. The system described herein may be used with any appropriate operating system.

Several implementations have been described. Nevertheless, it will be understood that various modifications may be made.

Accordingly, other implementations are within the scope of the following claims.

## Claims

1. A dialysis tubing set comprising:
at least one fluid line (802) configured to connect a dialysis fluid source and a pump of a dialysis machine; and
a flow chamber (806) connected to the at least one fluid line, **characterized in that** the flow chamber has a thermochromic material configured to change color when exposed to a specified temperature range, wherein the thermochromic material is configured as an insert (810) and is positioned within the at least one fluid line.

2. The dialysis tubing set of claim 1, wherein the thermochromic material is configured to remain visible to a user.

3. The dialysis tubing set of claim 1 or 2, wherein the dialysis tubing set is configured to be aligned with an optical sensor (304, 412, 702) of a dialysis machine.

4. The dialysis tubing of any of the above claims, wherein the thermochromic material comprises at least two thermochromic elements each configured to change color at a different temperature.

5. The dialysis tubing set of claim 4, wherein each thermochromic element is positioned on a different side of the flow chamber.

6. The dialysis tubing set of claim 4 or 5, wherein a first thermochromic component is positioned above a second thermochromic component.

7. The dialysis tubing set of claim 1, wherein the at least one fluid line comprises a first fluid path through a central channel of the insert and a second fluid path between the insert and the at least one fluid line.

8. The dialysis tubing set of any of the above claims, wherein the specified temperature range is between 35-39 °C.

9. A dialysis system comprising:
a dialysis machine having an optical sensor; and
the disposable tubing set according to any preceding claim, wherein the flow chamber is aligned with the optical sensor.

10. The dialysis system of claim 9, further comprising a light source (714) positioned to align with the flow chamber of the disposable tubing set.

11. The dialysis system of claim 9 or 10, wherein the flow chamber is positioned outside the dialysis machine.

12. The dialysis system of any of claims 9-11, wherein the optical sensor is a color sensor.

## Patentansprüche

1. Dialyseschlauchset, umfassend:
mindestens eine Fluidleitung (802), die ausgelegt ist, um eine Dialysefluidquelle und eine Pumpe einer Dialysemaschine zu verbinden; und
eine Durchflusskammer (806), die mit der mindestens einen Fluidleitung verbunden ist, **dadurch gekennzeichnet, dass** die Durchflusskammer ein thermochromes Material aufweist, das ausgelegt ist, um die Farbe zu verändern, wenn es einem bestimmten Temperaturbereich ausgesetzt wird, wobei das thermochrome Material als Einlage (810) ausgelegt ist und innerhalb der mindestens einen Fluidleitung positioniert ist.

2. Dialyseschlauchset nach Anspruch 1, wobei das thermochrome Material ausgelegt ist, um für einen Benutzer sichtbar zu bleiben.

3. Dialyseschlauchset nach Anspruch 1 oder 2, wobei das Dialyseschlauchset ausgelegt ist, um mit einem optischen Sensor (304, 412, 702) einer Dialysemaschine ausgerichtet zu werden.

4. Dialyseschlauch nach einem der vorhergehenden Ansprüche, wobei das thermochrome Material mindestens zwei thermochrome Elemente umfasst, die jeweils ausgelegt sind, um bei einer verschiedenen Temperatur die Farbe zu verändern.

5. Dialyseschlauchset nach Anspruch 4, wobei jedes thermochrome Element auf einer verschiedenen Seite der Durchflusskammer positioniert ist.

6. Dialyseschlauchset nach Anspruch 4 oder 5, wobei die erste thermochrome Komponente über einer zweiten thermochromen Komponente positioniert ist.

7. Dialyseschlauchset nach Anspruch 1, wobei die mindestens eine Fluidleitung einen ersten Fluidpfad durch einen Zentralkanal der Einlage hindurch und einen zweiten Fluidpfad zwischen der Einlage und der mindestens einen Fluidleitung umfasst.

8. Dialyseschlauchset nach einem der vorhergehenden Ansprüche, wobei der bestimmte Temperaturbereich zwischen 35 und 39 °C liegt.

9. Dialysesystem, umfassend:
eine Dialysemaschine mit einem optischen Sensor; und
das Einweg-Schlauchset nach einem der vorhergehenden Ansprüche, wobei die Durchflusskammer mit dem optischen Sensor ausgerichtet ist.

10. Dialysesystem nach Anspruch 9, des Weiteren umfassend eine Lichtquelle (714), die in Ausrichtung mit der Durchflusskammer des Einweg-Schlauchsets zu positionieren ist.

11. Dialysesystem nach Anspruch 9 oder 10, wobei die Durchflusskammer außerhalb der Dialysemaschine positioniert ist.

12. Dialysesystem nach einem der Ansprüche 9 bis 11, wobei der optische Sensor ein Farbsensor ist.

## Revendications

1. Ensemble tubulure pour dialyse comprenant :
au moins une ligne de fluide (802) conçue pour relier une source de fluide de dialyse et une pompe d'une machine de dialyse ; et
une chambre d'écoulement (806) raccordée à l'au moins une ligne de fluide, **caractérisé en ce que** la chambre d'écoulement présente un matériau thermochromique conçu pour changer de couleur lorsqu'il est exposé à une plage de température spécifiée, dans lequel le matériau thermochromique est conçu comme en tant qu'insert (810) et est positionné à l'intérieur de l'au moins une ligne de fluide.

2. Ensemble tubulure pour dialyse selon la revendication 1, dans lequel le matériau thermochromique est conçu pour rester visible par un utilisateur.

3. Ensemble tubulure pour dialyse selon la revendication 1 ou 2, dans lequel l'ensemble tubulure pour dialyse est conçu pour être aligné sur un capteur optique (304, 412, 702) d'une machine de dialyse.

4. Tubulure pour dialyse selon l'une quelconque des revendications précédentes, dans laquelle le matériau thermochromique comprend au moins deux éléments thermochromiques conçus chacun pour changer de couleur à une température différente.

5. Ensemble tubulure pour dialyse selon la revendication 4, dans lequel chaque élément thermochromique est positionné sur un côté différent de la chambre d'écoulement.

6. Ensemble tubulure pour dialyse selon la revendication 4 ou 5, dans lequel un premier composant thermochromique est positionné au-dessus d'un second composant thermochromique.

7. Ensemble tubulure pour dialyse selon la revendication 1, dans lequel l'au moins une ligne de fluide comprend un premier chemin de fluide à travers un canal central de l'insert et un second chemin de fluide entre l'insert et l'au moins une ligne de fluide.

8. Ensemble tubulure pour dialyse selon l'une quelconque des revendications précédentes, dans lequel la plage de température spécifiée est comprise entre 35 et 39 °C.

9. Système de dialyse comprenant :
une machine de dialyse comportant un capteur optique ; et
l'ensemble tubulure à usage unique selon une quelconque revendication précédente, dans lequel la chambre d'écoulement est alignée sur le capteur optique.

10. Système de dialyse selon la revendication 9, comprenant en outre une source de lumière (714) positionnée pour s'aligner sur la chambre d'écoulement de l'ensemble tubulure à usage unique.

11. Système de dialyse selon la revendication 9 ou 10, dans lequel la chambre d'écoulement est positionnée à l'extérieur de la machine de dialyse.

12. Système de dialyse selon l'une quelconque des revendications 9 à 11, dans lequel le capteur optique est un capteur de couleur.
